# EUROPÄISCHE PATENTSCHRIFT

(11) **EP 0 367 840 B1**
(45) Veröffentlichungstag und Bekanntmachung des Hinweises auf die Patenterteilung: **03.02.1993**
(21) Anmeldenummer: 88118478.2
(22) Anmeldetag: 05.11.1988
(51) Int. Cl.: A61K 35/16

(54) **Verfahren zur Herstellung eines hochreinen, nicht infektiösen Antihämophiliefaktors mittels Chromatographie**
Process for producing by chromatography a non-infectious antihemophilic factor with high purity
Procédé de préparation d'un facteur antihémophilique non infectieux et de grande pureté par chromatographie

(43) Veröffentlichungstag der Anmeldung: 16.05.1990
(73) Patentinhaber: Octapharma AG, 8750 Glarus (CH)
(72) Erfinder: Smith, Andrew, South Haven, Michigan 49090 (US)
(74) Vertreter: Werner, Hans-Karsten, Dr.Dipl.-Chem.

(56) Entgegenhaltungen:
- EP-A- 0 104 356
- EP-A- 0 173 242
- WO-A-86/04486
- DE-A- 2 715 832
- GB-A- 1 178 958
- US-A- 4 386 068
- THE BRITISH JOURNAL OF HAEMATOLOGY, Band 43, 1979, Seiten 669-674, Blackwell Scientific Publications; D.E.G. AUSTEN: "The chromatographic separation of factor VIII on aminohexyl sepharose"

## Beschreibung

Die Erfindung betrifft ein Verfahren zur Herstellung eines hochreinen, nicht infektiösen Antihämophiliefaktors (AHF oder Faktor VIII) aus Blutplasma durch Behandlung einer an Faktor VIII angereicherten Fraktion mit biokompatiblen organischen Lösungsmitteln/Detergenzien und anschließende weitere Reinigungsschritte.

In der EP-A-0 238 701 wird ein Verfahren zur Herstellung eines hochreinen, nicht infektiösen Antihämophiliefaktors beschrieben, wobei die vorbehandelten Fraktionen ein Kryopräzipitat sind, die mittels Ethanolfällung von Fibrinogen, Globulinen, Albuminen und anderen störenden Bestandteilen befreit werden. Die Anreicherung aus dem Kryopräzipitat ist notwendig, da Faktor VIII nur in äußerst geringen Mengen in dem Material enthalten ist. Dieser Anreicherungsschritt beeinträchtigt jedoch den Gehalt an AHF im Endprodukt. Die bisher bekannten Verfahren zur Herstellung von Faktor VIII ergeben nur geringe Mengen an wirksamer Substanz. Bei der Applikation des auf herkömmlichem Wege hergestellten Faktors VIII belastet man den Patienten daher mit großen Mengen antigener Substanzen. Diese Verfahrensweise ist nicht unbedenklich. Es hat daher nicht an Versuchen gefehlt, den Faktor VIII durch Trennungsoperationen weiter anzureichern. So wurde versucht, mittels Affinitätschromatographie mit gegen Faktor VIII gerichteten tierischen Antikörpern Produkte mit höherer spezifischer Aktivität zu erhalten. Diese Technik ist jedoch sehr aufwendig und kostenintensiv. Zum anderen ist sie auch deshalb nicht unbedenklich, da stets eine gewisse Menge tierischen Eiweißes bei jeder chromatographischen Trennung aus der Säule gewaschen wird.

In der Europäischen Patentanmeldung 88 108 458.6 wird vorgeschlagen, nach der Virusinaktivierung der Kryopräzipitation, eine chromatographische Trennung mit Ionenaustauschern durchzuführen. Dies führt zwar zu einer Ausbeutesteigerung bei der Herstellung von Faktor VIII, beinhaltet jedoch die Kryopräzipitation. Der Verfahrenssschritt der Kryopräzipitation ist nachteilig, weil eine quantitative Fällung des im Blutplasma vorhandenen Faktors VIII nicht erreicht wird, so daß hohe Ausbeuteverluste in Kauf genommen werden müssen.

Die EP-A-0 173 242 beschreibt ein Verfahren zur chromatographischen Gewinnung von Faktor VIII Präparationen an Anionenaustauschermaterialien, die ausschließlich auf Kohlenhydraten basieren. Die Kohlenhydratmatrix ist modifiziert mit DEAE-Gruppen. Es werden insbesondere DEAE-Sepharose sowie DEAE-Zellulose als geeignet beschrieben. Desweiteren wird in der EP-A-0 173 242 ausschließlich von Kryopräzipitat zur Präparation von Faktor VIII ausgegangen.

Die WO 86/04486 betrifft ein Verfahren zur Isolierung von Faktor VIII durch Chromatographie an anorganischen Materialien, nämlich Aluminiumoxid. Obligatorisch dabei ist die Anwesenheit von Zuckern, Alkoholen, Aminosäuren oder Salzen. Im übrigen geht diese Entgegenhaltung auch von Fraktionen, die aus Kryopräzipitat gewonnen wurden, aus.

Die GB-A-1,178,958 betrifft eine Reinigung des Faktors VIII, allerings wiederum aus Kryopräzipitat. In der GB-A-1,178,958 wird Faktor VIII aus Kryopräzipitat gewonnen durch Präzipitation mit Glycin, Präzipitation mit Polyethylenglykol. Es schließen sich dann verschiedene Zentrifugationsschritte an. Danach kann das erhaltene Präparat mittels einer ECTEOLA-Zellulosesäule behandelt werden. ECTEOLA Cellulose Resin ist modifizierte Zellulose, die aktive basische Substituenten in die Zellulose eingeführt enthält durch Reaktion von Epichlorhydrin und Triethanolamin.

Die DE-A-27 15 832 beschreibt die Gewinnung des Prothrombinkomplexes mittels DEAE-Dextranen. Dabei verbleibt Faktor VIII im Überstand. Dieses Verfahren betrifft somit eine adsorptive Vorreinigung des Blutplasmas für die Faktor VIII-Gewinnung.

Aus "The British Journal of Haematology", Band 43, 1979, Seiten 669 - 674 ist die Trennung von Faktor VIII an Aminohexyl substituierter Agarose beschrieben. Humanes Plasma wird einer säulenchromatographischen Trennung unterzogen. Agarose entspricht jedoch dem in der EP-A-0 173 242 beschriebenen chromatographischen Material.

Die EP-A-0 104 356 betrifft die Reinigung von Faktor VIII mittels Gelpermeations-Chromatographie.

Die der Erfindung zugrunde liegende Aufgabe besteht in der Bereitstellung eines Verfahrens zur Herstellung eines hochreinen, durch Behandlung mit biokompatiblen organischen Lösungsmitteln/Detergenzien nicht infektiösen Antihämophiliefaktors (AHF oder Faktor VIII) aus Blutplasma in höherer Ausbeute und mit höherer biologischer Aktivität pro Gewichtseinheit Protein. Eine weitere Aufgabe besteht darin, die Kryopräzipitation zu vermeiden.

Diese Aufgabe kann in überraschend einfacher Weise dadurch gelöst werden, daß die an Faktor VIII angereichte Fraktion durch eine chromatographische Trennung an einem hydrophilen Chromatographiematerial auf Basis von Copolymerisaten aus Oligoethylenglycolen, Glycidylmethacrylaten und Pentaerythroldimethacylaten, wobei das Chromatographiematerial mit Ionenaustauschergruppen modifiziert ist, gewonnen wird.

Vorteilhaft am erfindungsgemäßen Verfahrens ist es, daß eine höhere Ausbeute an Faktor VIII erzielt wird, indem man anstelle der Kryopräzipitation nach dem Stand der Technik die Gelpermeationschromatographie mit dem oben genannten Ionenaustauschermaterialien durchführt. Dabei wird gefrorenes Plasma in einem Wasserbad aufgetaut. Sobald eine gewisse Temperatur, die vorzugsweise 25°C beträgt, erreicht ist, wird das Plasma, vorzugsweise mit 50 vol.-% Wasser, verdünnt und filtriert.

Danach wird das so behandelte Plastma einer Gelpermeaionschromatographie auf Ionenaustauscherbasis, wie Fractogen®-DEAE-Harzen unterworfen. Fractogel® ist ein hydrophiles Chromatographiematerial auf Basis von Copolymerisaten aus Oligoethylenglycolen, Glycidylmethacrylaten in der Säule wird vorher mit einem Glycin Natriumcitrat, Natriumchlorid und Heparin enthaltenden Puffer äquilibriert. Vorzugsweise hat der Puffer die folgende Zusammensetzung: 10 - 50 mM Natriumchlorid, 2 - 18 mM Natriumcitrat, 30 - 210 mM Glycin und 20 - 180 U/L Heparin. Die mit diesem Puffer erzielten Ionenstärkebedingungen führen dazu, daß der Faktor VIII an dem Säulenmaterial adsorbiert wird. Andere Blutplasmabestandteile werden getrennt, indem mit einigen Säulenvolumina des Auftragspuffers bei einem pH-Wert zwischen 6,5 und 6,9 gewaschen wird.

Danach ändert man die Pufferbedingungen durch Erhöhung der Salzkonzentration, vorzugsweise der Kochsalzkonzentration. Dies kann kontinuierlich oder auch diskontinuierlich geschehen. Vorzugsweise erhöht man die Natriumchloridkonzentration in dem oben angegebenen beispielhaften Puffersystem in drei Schritten über 20 - 80 mM, 30 - 180 mM und 40 - 360 mM. Das Säulenmaterial wird mehrfach mit den Puffern höherer Salzkonzentration gewaschen, wobei Verunreinigungen entfernt werden. Der Faktor VIII eluiert dann bei hohen Ionenstärken.

Die Faktor VIII enthaltende Fraktion wird aufgefangen und durch Hinzufügen von humanem Albumin stabilisiert. Vorzugsweise beträgt die Konzentration an humanem Serumalbumin (HSA) 1 mg/ml. Danach kann die Lösung mit Aluminiumhydroxid behandelt werden, um Restmengen von Prothrombinkomplex-Faktoren zu beseitigen. Danach wird filtriert.

Die Virusinaktivierung erfolgt gemäß der Methode, die in der EP-A 131 740 beschrieben wird, durch Behandlung mit biokompatiblen organischen Lösungsmitteln/Detergenzien, vorzugsweise Tween/TNBP (Tri-n-Butylphosphat). Gute Ergebnisse werden auch mit Natriumcholat/TNBP erzielt.

In einer weiteren vorteilhaften Ausgestaltung der Erfindung wird eine höhere Ausbeute an Faktor VIII mit höherer spezifischer Aktivität erzielt, indem nach der Entfernung der Viren die Probe einer weiteren Gelpermeationschromatographie an Ionenaustauschermaterialien unterworfen wird.

Nach der Virusinaktivierung kann die Rohfraktion verdünnt und filtriert werden.

Woods, K. und Orme, Th. beschreiben in der EP-A-0 239 859, daß es von Vorteil ist, nach der Virusentfernung oder -inaktivierung und vor der chromatographischen Trennung die Probe mit Ölen zu extrahieren, vorzugsweise mit Sojaöl, Rizinöl und/oder Baumwollsamenöl.

Für den Fall, daß als Ausgangsmaterial nicht aufgetautes oder frisches Blutplasma verwendet wird, sondern handelsübliches Kryopräzipitat, wird vorzugsweise wie folgt vorgegangen: handelsübliches Kryopräzipitat wird bei Raumtemperatur innerhalb von 3 bis 4 Stunden aufgetaut und in Stücke von ca. 1 bis 2 cm zerteilt. Diese Stücke werden bei Temperaturen zwischen 10 und 25°C durch Rühren suspendiert in etwa dem doppelten Volumen Wasser, welches 1 bis 3 U/ml Heparin-Natrium enthält. Die Suspension wird mit 0,1 M Essigsäure auf einen pH-Wert von mindestens 7,0 bis 8, vorzugsweise 7,0 bis 7,1 eingestellt. Es wird 15 bis 60 Minuten, vorzugsweise 30 Minuten bei Raumtemperatur gerührt. Daraufhin werden etwa 108 g 2% Aluminiumhydroxid-Suspension pro kg Kryopräzipitat hinzugefügt und 1 bis 10 Minuten, vorzugsweise 5 Minuten bei Raumptemperatur gerührt. Mit Säure, vorzugsweise 0,1 M Essigsäure, wird der Säuregehalt auf pH 6,0 bis 7,0, vorzugsweise 6,5 bis 6,6 eingestellt. Die Probe wird auf 10 bis 18°C, vorzugsweise 14 bis 16°C gekühlt. Man zentrifugiert bei dieser Temperatur, beispielsweise in einer Sharpes AS-16 (Cepa 61)-Zentrifuge mit einer Rate von 1,0 L/min. Danach schließt sich eine Filtration des Überstandes, beispielsweise durch ein Pall AB-1 UO10ZP-Filter an. Vorzugsweise nach dem Abzentrifugieren und Filtrieren erfolgt eine Virusinaktivierung. Besonders bewährt hat sich die Virusinaktivierung mit Hilfe von Tween/TNBP (Tri-n-Butylphosphat). Gute Ergebnisse werden auch mit Natriumcholat/TNBP erzielt. Das Tween/TNBP bzw. Natriumcholat/TNBP-Gemisch kann beispielsweise durch Ölexraktion wieder entfernt werden.

Nach der Virusinaktivierung gibt man die Probe auf eine Chromatographiesäule, die ein Gelpermeationsmaterial mit Ionenaustauscheraktivität aufweist, wie beispielsweise Fractogel®-DEAE. Die Säulenkapazität soll vorzugsweise so beschaffen sein, daß 0,5 kg des Säulenmaterials pro kg Kryopräzipitat sich in der Säule befinden. Die Säule wird mit der Probe beschickt und mit Puffern gewaschen. Nach Elution der Probe mit einem Puffer höherer Ionenstärke wird das erhaltene Produkt mit einem Puffer mit niedrigem Salzgehalt verdünnt und, falls nötig, auf pH 6,5 bis 7,45, vorzugsweise 6,9 bis 7,1 eingestellt. Danach wird erneut filtriert, vorzugsweise an Nitrocellulosefiltern, worauf sich eine Sterilfiltration anschließt.

Durch die erfindungsgemäßen Maßnahmen ist es erstmals möglich, in hohen Ausbeuten einen hochreinen Antihämophiliefaktor herzustellen, der eine bisher nicht erreichte spezifische Aktivität aufweist. Die bisherigen Ergebnisse deuten darauf hin, daß durch die bisher übliche Ethanolfällung oder Kryopräzipitation ein beträchtilicher Anteil des Faktors VIII irreversibel zerstört wird.

Das Verfahren wird anhand der folgenden Beispiele näher erläutert.

### Beispiel 1

Frisches oder tiefgefrorenes humanes Plasma wird, gegebenenfalls in einem Wasserbad, temperiert, bis die Mischung eine Temperatur von 20°C erreicht. Das Plasma wird mit 50 vol.-% Wasser verdünnt und danach filtriert. Das Plasmafiltrat wird in eine Ionenaustauschersäule mit Fractogen®-DEAE-Harz aufgetragen. Die Säule wurde zuvor mit einem Puffer folgender Zusammensetzung äquilibriert:

| | |
|---|---|
| 50 mM | Natriumchlorid |
| 10 mM | Natriumcitrat |
| 120 mM | Glycin |
| 100 U/L | Heparin pH 6,5 bis 6,9 |

Nach dem Auftragen der Probe wird das Ionenaustauscherharz mit dem Waschpuffer mehrere Male gewaschen. Danach wird schrittweise die Konzentration an Kochsalz erhöht, indem zunächst mit einem Puffer gewaschen wird, der 100 mM Natriumchlorid, danach 160 mM Natriumchlorid gefolgt von 200 mM Natriumchlorid enthält. Die Faktor VIII enthaltenen Fraktionen werden gesammelt und durch Zugabe von 1,0 mg/ml humanem Serumalbumin stabilisiert. Die weitere Bearbeitung erfolgt analog den folgenden Beispielen, nur daß anstelle des dort verwendeten handelsüblichen Kryopräzipitats, die Faktor VIII enthaltenden Säulenfraktionen eingesetzt werden.

### Beispiel 2

### Anreicherung des Faktor VIII

Handelsübliches Kryopräzipitat wird bei Raumtemperatur innerhalb von 3 bis 4 Stunden aufgetaut und in Stücke von ca. 1 bis 2 cm zerteilt. Diese Stücke werden bei 20 bis 25°C durch Rühren suspendiert und in etwa dem doppelten Volumen Wasser, welches 2 U/ml Heparin-Natrium enthält, suspendiert. Die Suspension wird mit 0,1 M Essigsäure auf einen pH-Wert von 7,0 bis 7,1 eingestellt. Man rührt 30 Minuten bei 20 bis 25°C. 108 g 2% Aluminiumhydroxid-Suspension pro kg Kryopräzipitat werden hinzugefügt und weitere 5 Minuten bei Raumptemperatur gerührt. Danach stellt man den pH-Wert mit 0,1 M Essigsäure auf pH 6,5 bis 6,6 ein. Danach kühlt man die Probe auf 14 bis 16°C ab, zentrifugiert in einer Sharples AS-16 (Cepa 61)-Zentrifuge mit einer Rate von 1,0 L/min. Der Überstand wird durch ein Pall AB-1 Uo10ZP-Filter filtriert.

### Beispiel 3

Vorbereitung der Chromatographiesäule für die Chromatographie nach der Befreiung der Rohfraktion von Viren.

Zur Trennung des extrahierten Kryopräzipitats wird eine Säule mit mindestens 0,5 l Fractogel®-DEAE Harz pro kg Kryopräzipitat verwendet. Die Säulenhöhe soll ≦ Durchmesser sien. Nach dem Beschicken der Säule mit Harz wird die Chromatographiesäule mit 5 Volumina 0,1 M Natriumchloridlösung gewaschen. Danach wäscht man mit einem Puffer A, der wie folgt zusammengesetzt ist:
110 mM Natriumchlorid, 10 mM Natriumcitrat-5H₂O, 120 mM Glycin, 1 mM Calciumchlorid-2H₂O, pH-Wert 6,9 bis 7,0, mit 1 M HCl einzustellen.

Sämtliche Puffer müssen virusfrei sein, da die folgenden Operationen mit virusfreien Extrakten des Kryopräzipitats durchgeführt werden.

### Beispiel 4

Die Probe wird auf die Säule aufgetragen und die Absorption des Durchflusses bei einer Wellenlänge von 280 nm beobachtet. Das Filtrat wird aufgenommen und auf Faktor VIII-Aktivität untersucht, ebenso wie das Produkt vor der Säulentrennung. Danach wird die Säule mit dem Puffer A gewaschen, bis die Absorption wieder ihren Ausgangswert erreicht. Danach wird die Säule mit Puffer B gewaschen. Dies geschieht so lange, bis die Absorption wieder die Grundlinie erreicht hat.

Der Puffer B hat folgende Zusammensetzung:
160 mM Natriumchlorid, 10 mM Natriumcitrat-5H₂O, 120 mM Glycin, 1 mM Calciumchlorid-2H₂O, pH-Wert 6,9 bis 7,0.

Die Elution des Produkts erfolgt mit dem Puffer C. Die nach Zugabe des Puffers C erscheinende Proteinfraktion wird gesammelt. Puffere C weist folgende Zusammensetzung auf:
250 mM Natriumchlorid, 20 mM Natriumcitrat-5H₂O, 80 mM Glycin, 16 mM Lysin, 2,5 mM Calciumchlorid-2H₂O, pH-Wert 6,9 bis 7,0.

Nach Elution der gewünschten Fraktion wird die Säule anschließend mit 5 Volumina des Puffers D, welcher 1 M Natriumchloridlösung enthält, gewaschen.

Die Regenerierung der Säule erfolgt durch Waschen mit 0,1 N NaOH (3 Säulenvolumina), gefolgt durch Waschen der Säule mit 0,1 N Salzäure (3 Säulenvolumina) und Waschen der Säule mit 5 Säulenvolumina 25% Alkohol in Wasser.

### Beispiel 5

Die gesammelten Fraktionen werden mit Puffer E, bestehend aus
20 mM Natriumcitrat, 80 mM Glycin, 16 mM Lysin, 2,5 mM Calciumchlorid-2H₂O, pH-Wert 6,9 bis 7,1, so lange verdünnt, bis sie eine Aktivität von 26 oder 35 U/ml aufweisen. Danach wird der pH-Wert, falls nötig, auf 6,9 bis 7,1 eingestellt, worauf sich eine Filtration des Produkts durch 0,45 µm Sealklean-Filter anschließt. Abschließend wird eine weitere Sterilfiltration durchgeführt.

## Patentansprüche

1. Verfahren zur Herstellung eines hochreinen, nicht infektiösen Antihämophiliefaktors (AHF oder Faktor VIII) aus Blutplasma durch Behandlung einer an Faktor VIII angereicherten Fraktion mit biokompatiblen organischen Lösungsmitteln/Detergenzien und anschließende weitere Reinigungsschritte, dadurch gekennzeichnet, daß die an Faktor VIII angereicherte Fraktion durch eine chromatographische Trennung an einem hydrophilen Chromatographiematerial auf Basis von Copolymerisaten aus Oligoethylenglycolen, Glycidylmethacrylaten und Pentaerythroldimethacrylaten, wobei das Chromatographiematerial mit Ionenaustauschergruppen modifiziert ist, gewonnen wird.

2. Verfahren nach Anspruch 1, dadurch gekennzeichnet, daß das Ionenaustauschmaterial ein mit DEAE-Gruppen modifiziertes Material ist.

3. Verfahren nach einem der Ansprüche 1 oder 2, dadurch gekennzeichnet, daß die Faktor VIII enthaltende Fraktion mit einer Aluminiumhydroxid-Suspension versetzt und nach Abkühlung von 10 bis 18°C, pH < 7 zentrifugiert oder filtriert wird.

4. Verfahren nach einem der Ansprüche 1 bis 3, dadurch gekennzeichnet, daß die Aufarbeitung der Faktor VIII enthaltenden Fraktion durch ein weiteres chromatographisches Verfahren erfolgt.

5. Verfahren nach Anspruch 4, dadurch gekennzeichnet, daß das weitere chromatographische Verfahren ein gelpermeationschromatographisches Verfahren auf Basis von Ionenaustauschern ist.

6. Verfahren nach Anspruch 4 oder ,5 dadurch gekennzeichnet, daß das Chromatographiematerial ein Anionenaustauschermaterial, wie DEAE-modifiziertes Material, ist.

## Claims

1. A process for the preparation of a highly pure non-infectious antihaemophilic factor (AHF or factor VIII) from blood plasma by treating a fraction wherein the factor VIII has been accumulated with biologically compatible organic solvents/detergents, followed by further purification steps, characterized in that the fraction wherein the factor VIII has been accumulated is recovered by means of a chromatographic separation on a hydrophilic chromatography material based on copolymers of oligoethyleneglycols, glycidyl methacrylates and pentaerythrol dimethacrylates, the chromatography material having been modified with ion exchanger groups.

2. The process according to claim 1, characterized in that the ion exchanger material is a material modified with DEAE groups.

3. The process according to either of claims 1 or 2, characterized in that the fraction containing the factor VIII is admixed with an aluminum hydroxide suspension and after cooling is centrifuged or filteres at from 10 °C to 18 °C and a pH <7.

4. The process according to anyone of claims 1 to 3, characterized in that the workup of the fraction containing the factor VIII is carried out by means of a further chromatographic process.

5. The process according to claim 4, characterized in that the further chromatographic process is a gel permeation chromatography process based on ion exchangers.

6. The process according to claims 4 or 5, characterized in that the chromatography material is an anion exchanger material such as a DEAE-modified material.

## Revendications

1. Procédé d'obtention d'un facteur anti-hémophilie (AHF ou facteur VIII), de haute pureté, non infectieux, à partir de plasma sanguin, par un traitement d'une fraction enrichie en facteur VIII avec des solvants/détergents organiques biocompatibles, immédiatement suivi d'étapes de purification, caractérisé en ce que la fraction enrichie en facteur VIII est obtenue par une séparation sur un matériau de chromatographie hydrophile, à base de copolymérisats d'oligométhylène-glycol, de méthacrylate de glycidyle et de diméthylacrylate de pentaérythrol, le matériau de chromatographie étant modifié par des groupes échangeurs d'ions.

2. Procédé selon la revendication 1, caractérisé en ce que le matériau échangeur d'ions est une matière modifiée par des groupes DEAE.

3. Procédé selon l'une des revendications 1 ou 2, caractérisé en ce que la fraction contenant du facteur VIII est mélangée avec une suspension d'hydroxyde d' aluminium et centrifugée ou filtrée après refroidissements de 10 à 18°C, à pH < 7.

4. Procédé selon l'une des revendications 1 à 3, caractérisé en ce que le retraitement de la fraction contenant du facteur VIII est opéré par un autre procédé chromatographique.

5. Procédé selon la revendication 4, caractérisé en ce que l'autre procédé chromatographique est un procédé de perméation sur gel à base d'échangeurs d'ions.

6. Procédé selon la revendication 4 ou 5, caractérisé en ce que le matériau de chromatographie est un matériau échangeur d'anions, telle qu'une matière modifiée par du DEAE.
